# EUROPÄISCHE PATENTSCHRIFT

(11) **EP 3 939 552 B1**
(45) Veröffentlichungstag und Bekanntmachung des Hinweises auf die Patenterteilung: **12.07.2023**
(21) Anmeldenummer: 21186491.3
(22) Anmeldetag: 19.07.2021
(51) Int. Cl.: A61F 5/01

(54) **EINBEINSCHIENE ZUR BEHANDLUNG VON KLUMPFÜSSEN UND ANDEREN FUSSDEFORMITÄTEN**
UNILATERAL ORTHOTIC FOR THE TREATMENT OF CLUB FOOT AND OTHER FOOT DEFORMITIES
GOUTTIÈRE DE JAMBE DESTINÉE AU TRAITEMENT DES PIEDS-BOTS ET D'AUTRES DIFFORMITÉS DU PIED

(30) Priorität: 17.07.2020 DE 102020119025
(43) Veröffentlichungstag der Anmeldung: 19.01.2022
(73) Patentinhaber: Johannsen, Petra, 78467 Konstanz (DE)
(72) Erfinder:
(74) Vertreter: Hauck Patentanwaltspartnerschaft mbB

(56) Entgegenhaltungen:
- US-A- 5 382 225
- US-A- 6 036 665
- US-A1- 2018 271 691

## Beschreibung

Die Erfindung betrifft eine Einbeinschiene zur Behandlung von Klumpfüßen und anderen Fußdeformitäten Die Erfindung ist in den Ansprüchen definiert.

Bei der Ponseti-Methode zur Korrektur des Klumpfußes wird zunächst durch Gipsen eine Korrektur herbeigeführt und die korrigierte Fehlstellung durch die Anwendung einer Schiene gehalten, damit kein Rezidiv auftritt. Hierbei werden die Füße mittels einer Stange in einer nach außen rotierten Stellung gehalten. Nach der Methode wird die Schiene über drei Monate nach Beendigung der Gipsregressionstherapie in Vollzeit angelegt und dann über vier Jahre nur in der Nacht. Das Fixieren beider Füße mittels der Schiene ist für Kinder und Eltern unangenehm, insbesondere wenn die Kinder älter werden.

Die EP 1 998 726 B1 beschreibt eine Orthese zur Klumpfußbehandlung, die die Belastungen des Patienten mindert und dennoch die Füße des Patienten in der gewünschten Ausrichtung fixiert, indem sie ganz oder teilweise elastisch ausgebildet ist. Somit kann die Orthese bei Belastung durch den Patienten etwas nachgeben, wodurch die Bewegungseinschränkung des Patienten beim Tragen der Orthese gemindert wird.

Die DE 10 2012 006 261 B4 beschreibt eine die Belastung des Patienten mindernde und die Füße in der gewünschten Ausrichtung haltende Orthese zur Klumpfußbehandlung, bei der Mittel zum lösbaren Halten der Füße eines Patienten an Gelenken an den beiden Enden eines Verbindungsträgers gehalten und in die Gelenke Federn integriert sind.

Die EP 2 637 612 B1 beschreibt orthopädische Fußgelenkvorrichtungen, die jeweils nur an einem einzigen Bein befestigt werden und daher dem Träger eine größere Bewegungsfreiheit ermöglichen. Diese Einbeinschienen weisen zwischen einem ersten Teil zur Verbindung mit einem Bein und einem zweiten Teil zur Verbindung mit einem Fuß einen Verbinder auf. Der Verbinder umfasst eine erste Einstellvorrichtung, die eine mit der Einstellung verbundene Relativbewegung des ersten Teils und des zweiten Teils um eine erste Vorrichtungsdrehachse herum gestattet. Ferner umfasst der Verbinder eine zweite Einstellvorrichtung, welche eine zweite mit der Einstellung verbundene Relativbewegung des ersten Teils und des zweiten Teils um eine zweite Vorrichtungsdrehachse herum gestattet. Die erste Vorrichtungsdrehachse entspricht im Wesentlichen einer dominanten anatomischen Drehachse des subtalaren Gelenks und die zweite Vorrichtungsdrehachse entspricht im Wesentlichen einer dominanten anatomischen Drehachse des tibiotalaren Gelenks. Die erste Einstellvorrichtung umfasst ein erstes Vorspannmittel zum Bereitstellen einer ersten Stützkraft zum Abstützen bei Verwendung des subtalaren Gelenks eines Benutzers in einer abduzierten, neutralen oder adduzierten Position. Die zweite Einstellvorrichtung umfasst ein zweites Vorspannmittel zum Bereitstellen einer zweiten Stützkraft zum Abstützen bei Verwendung des tibiotalaren Gelenks eines Benutzers in einer dorsalflektierten, neutralen oder plantarflektierten Position. Die Einbeinschiene erlaubt Bewegungen aus dem pathologischen Bereich heraus bis in den überkorrigierten Bereich, sodass das Kind den Fuß wieder in die Klumpfuß-Stellung bringen kann, was ihm zwar eine vermehrte Beweglichkeit verschafft, in therapeutischer Hinsicht jedoch fragwürdig erscheint. Die Bewegung um die zweite Vorrichtungsdrehachse weicht von der Außenrotation ab und ist aufwendig in der baulichen Umsetzung.

Die US 6,036,665 beschreibt eine orthopädische Vorrichtung zum Festlegen oder Korrigieren des Unterschenkels, Fußgelenks und von Fußdeformitäten, die leicht an den Behandlungsfortschritt durch die korrigierenden Kräfte auf die verformten Körperteile anpassbar ist. Sie umfasst eine Mehrzahl gelenkig miteinander verbundener Teile und diese miteinander verbindende Befestigungselemente. Von Zeit zu Zeit können die Befestigungselemente gelöst werden, die Teile der Vorrichtung relativ zueinander in andere Positionen bewegt und die Befestigungselemente wieder festgesetzt werden, um neue korrigierende Biegemomente oder Drehmomente auf Teile des Körpers auszuüben. Die Vorrichtung umfasst gelenkig miteinander verbundene Teile zum Ausüben von Biegemomenten auf den Unterschenkel, um eine nach innen oder nach außen gekrümmte Lage zu korrigieren, und zum Belasten des Fußes so, dass die Zehen nach oben oder nach unten, nach innen oder nach außen geschwenkt werden.

Die US 2018/0271691 A1 beschreibt eine Gehunterstützungsvorrichtung, die ein unangenehmes Tragegefühl vermeiden soll, indem sie sämtliche Bewegungen des Fußgelenkes zulässt, insbesondere die Pronation und Supination. Ein Adduktions/Abduktions-Mechanismus weist eine bogenförmige Führung mit der Adduktions/Abduktions-Achse 13 im Zentrum auf. Infolgedessen behindert der Mechanismus nicht die Bewegung des Benutzers, so wie das Gehen. Zudem kann der Mechanismus mit einer einfachen Struktur und hoher Tragesteifigkeit ausgeführt werden.

Davon ausgehend liegt der Erfindung die Aufgabe zugrunde, eine dem Kind Bewegungsfreiräume verschaffende Einbeinschiene zu schaffen, welche die Therapie nach der Ponseti-Methode noch besser unterstützt und die bauliche Umsetzung erleichtert.

Die Aufgabe wird durch eine Einbeinschiene mit den Merkmalen von Anspruch 1 gelöst. Vorteilhafte Ausführungsarten der Einbeinschiene sind in Unteransprüchen angegeben.

Die erfindungsgemäße Einbeinschiene zur Behandlung von Klumpfüßen und anderen Fußdeformitäten umfasst eine erste Halteeinrichtung zum lösbaren Verbinden mit einem Bein eines Trägers, eine zweite Halteeinrichtung zum lösbaren Verbinden mit einem Fuß an demselben Bein des Trägers und eine Verbindungseinrichtung zwischen der ersten Halteeinrichtung und der zweiten Halteeinrichtung, wobei die Verbindungseinrichtung ein erstes Drehgelenk mit einer ersten Drehachse umfasst, die der Drehachse des oberen Sprunggelenkes entspricht, und die Verbindungseinrichtung ein zweites Drehgelenk mit einer vertikal ausgerichteten zweiten Drehachse umfasst, wodurch das erste Drehgelenk den Fuß eines Trägers bei einer Dorsalextension und das zweite Drehgelenk den Fuß eines Trägers bei einer Außenrotation führt.

Die erfindungsgemäße Einbeinschiene führt den Fuß des Trägers bzw. Kindes in eine zum Körper angehobene und in eine nach außen rotierte Stellung. Gegenüber einer statischen Schiene, welche jeden Fuß in einer festen Stellung festhält, ermöglicht sie eine Bewegung des Fußes, sodass die Kapseln, Sehnen usw. gedehnt werden und nicht steif und unbeweglich bleiben. Zugleich werden dem Träger in einem akzeptierbaren Bereich Freiräume für die Bewegung geschaffen. Die erste Drehachse des ersten Drehgelenks entspricht der Drehachse des oberen Sprunggelenkes, das ein einfaches Gelenk mit einer Drehung nur um eine einzige Achse ist. Die zweite Drehachse des zweiten Drehgelenks ist vertikal ausgerichtet, um eine Außenrotation bzw. Abduktion des Fußes gemäß der Ponseti-Methode durchzuführen. Die Beweglichkeit des Fußes lässt diese Bewegung zu. Das zweite Drehgelenk kann baulich einfacher und platzsparender als ein Drehgelenk mit einer Vorrichtungsdrehachse entsprechend der anatomischen Drehachse des tibiotalaren Gelenks ausgeführt werden. Die Einbeinschiene verbessert die Anwendung der Ponseti-Methode und ist baulich einfacher und platzsparender ausführbar.

Gemäß einer Ausführungsart der Erfindung umfasst das erste Drehgelenk eine vorgespannte erste Federeinrichtung, die das erste Drehgelenk in Richtung der Ausführung einer Dorsalextension eines mit der zweiten Halteeinrichtung verbundenen Fußes belastet und/oder umfasst das zweite Drehgelenk eine vorgespannte zweite Federeinrichtung, die das zweite Drehgelenk in Richtung der Ausführung einer Außenrotation eines mit der zweiten Halteeinrichtung verbundenen Fußes belastet. Durch die erste Federeinrichtung und/oder die zweite Federeinrichtung wird der Fuß unter Vorspannung in die Dorsalextension und/oder Außenrotation gedrückt. Hierbei werden die Sehnen und Kapseln in einer für das Kind angenehmen Weise gedehnt, weil die Füße nicht starr durch eine Schiene miteinander verbunden sind. Insbesondere wird der Fuß in die Überkorrekturstellung gedrückt, wenn die Körperspannung nachlässt, weil das Kind schläft oder abgelenkt ist. Will sich das Kind jedoch plötzlich bewegen, so ist dies entgegen der Wirkung der ersten Federeinrichtung und/oder der zweiten Federeinrichtung möglich.

Gemäß einer weiteren Ausführungsart ist die erste Federeinrichtung eine erste Federeinrichtung mit einstellbarer Vorspannung und/oder ist die zweite Federeinrichtung eine zweite Federeinrichtung mit einstellbarer Vorspannung. Die Einstellbarkeit ermöglicht eine Anpassung der Vorspannung an das Alter und die Resistenz des jeweiligen Trägers.

Gemäß einer weiteren Ausführungsart ist die erste Federeinrichtung eine erste Spiralfeder und/oder ist die zweite Federeinrichtung eine zweite Spiralfeder. Hierdurch wird eine platzsparende Unterbringung in dem ersten Drehgelenk und/oder dem zweiten Drehgelenk ermöglicht. Ferner kann hierdurch die Federkraft über große Drehwinkel ausgeübt werden. Gemäß einer weiteren Ausführungsart ist die erste Spiralfeder eine erste Flachbandfeder und/oder ist die zweite Spiralfeder eine zweite Flachbandfeder.

Gemäß einer weiteren Ausführungsart umfasst das erste Drehgelenk ein erstes Zahnrad mit einem damit verbundenen zentralen ersten Außenmehrkant und einer ersten Lagerbohrung, eine in die erste Lagerbohrung und in weitere erste Lagerbohrungen der beiden ersten Gelenkteile des ersten Drehgelenkes eingesetzte erste Drehachse, weist die erste Spiralfeder ein auf dem ersten Außenmehrkant formschlüssig gehaltenes erstes Federende und ein an einem ersten Gelenkteil gehaltenes zweites Federende auf und ist in eine erste Gewindebohrung in dem anderen ersten Gelenkteil eine mit dem ersten Zahnrad kämmende erste Madenschraube mit einem ersten Werkzeugangriff eingeschraubt, wodurch mittels eines an den ersten Werkzeugangriff angesetzten Werkzeugs das erste Zahnrad drehbar und die erste Spiralfeder in unterschiedlichem Ausmaß vorspannbar ist. Gemäß einer weiteren Ausführungsart umfasst das zweite Drehgelenk ein zweites Zahnrad mit einem damit verbundenen zentralen zweiten Außenmehrkant und einer zweiten Lagerbohrung, eine in die zweite Lagerbohrung und in weitere zweite Lagerbohrungen der beiden zweiten Gelenkteile des zweiten Drehgelenkes eingesetzte zweite Drehachse, weist die zweite Spiralfeder ein auf dem zweiten Außenmehrkant formschlüssig gehaltenes erstes Federende und ein an einem zweiten Gelenkteil gehaltenes zweites Federende auf und ist in eine zweite Gewindebohrung in dem anderen zweiten Gelenkteil eine mit dem zweiten Zahnrad kämmende zweite Madenschraube mit einem zweiten Werkzeugangriff eingeschraubt, wodurch mittels eines an den zweiten Werkzeugangriff angesetzten Werkzeugs das zweite Zahnrad drehbar und die zweite Spiralfeder in unterschiedlichem Ausmaß vorspannbar ist. Hierdurch wird bei platzsparender Unterbringung und langen Federwegen eine einfache Einstellung der Vorspannung der jeweiligen Federeinrichtung ermöglicht. Die Selbsthemmung des durch Madenschraube und Zahnrad gebildeten Schneckengetriebes unterbindet eine ungewollte Verstellung der eingestellten Vorspannung.

Gemäß einer weiteren Ausführungsart umfasst das erste Drehgelenk erste Drehwinkelbegrenzungen, welche ein Drehen des ersten Drehgelenkes begrenzen, sodass ein mit der zweiten Halteeinrichtung verbundener Fuß an einem mit der ersten Halteeinrichtung verbundenen Bein zwischen einer Neutralstellung und einer Dorsalflexion mit einem Winkel von maximal 55 bis 30°, vorzugsweise von maximal 45° drehbar ist und/oder umfasst das zweite Drehgelenk zweite Drehwinkelbegrenzungen, welche die Drehbewegung des zweiten Drehgelenkes begrenzen, sodass ein mit der zweiten Halteeinrichtung verbundener Fuß an einem mit der ersten Halteeinrichtung verbundenen Bein zwischen einer Stellung parallel zur Medianebene und einer Abduktion mit einem Winkel von maximal 75 bis 50°, vorzugsweise von maximal 65° zur Medianebene bewegbar ist. Hierdurch wird eine Dorsalextension und eine Außenrotation im therapeutischen Bereich erreicht. Die Neutralstellung ist die Stellung, die der Fuß einnimmt, wenn er gerade auf einen ebenen Boden aufgesetzt ist. Die Drehwinkelbegrenzungen verhindern, dass der Fuß im pathologischen Bereich bewegt wird und in die Klumpfuß-Stellung gelangt.

Gemäß einer weiteren Ausführungsart ist eine erste Feststelleinrichtung vorhanden, die ausgebildet ist, die erste Federeinrichtung in mindestens einer Position festzustellen und/oder ist eine zweite Feststelleinrichtung vorhanden, die ausgebildet ist, die zweite Federeinrichtung in mindestens einer Position festzustellen. Gemäß einer weiteren Ausführungsart sind die erste Feststelleinrichtung und ist die zweite Feststelleinrichtung ausgebildet, die erste Federeinrichtung und die zweite Federeinrichtung in Positionen festzustellen, in denen die erste Halteeinrichtung und die zweite Halteeinrichtung ein Bein und den zugehörigen Fuß in der Normalstellung zu halten. Durch Feststellen der ersten Feststelleinrichtung und der zweiten Feststelleinrichtung kann der Komfort beim Anziehen der Einbeinschiene und die korrekte Ausrichtung der Einbeinschiene verbessert werden.

Gemäß einer weiteren Ausführungsart weist die erste Halteeinrichtung eine das Schienbein und zumindest ein Teil der Waden einfassende Schale auf. Gemäß einer weiteren Ausführungsart ist die erste Halteeinrichtung ausgewählt aus einer Range von ersten Halteeinrichtungen mit unterschiedlich großen Schalen für unterschiedlich große Beine. Hierdurch wird eine einfache Anpassung der Einbeinschiene an unterschiedliche Körpergrößen ermöglicht. Gemäß einer weiteren Ausführungsart weist die erste Halteeinrichtung mindestens einen ersten Riemen mit einem ersten Riemenverschluss auf, der ausgebildet ist, am Bein eines Trägers fixiert zu werden.

Gemäß einer weiteren Ausführungsart weist die zweite Halteeinrichtung eine Aufnahmeplatte zum Unterstützen des Fußes auf. Gemäß einer weiteren Ausführungsart weist die zweite Halteeinrichtung oberhalb der Aufnahmeplatte eine an der Aufnahmeplatte um eine horizontale Achse kippbar gelagerte Kippplatte auf, auf der der Fuß aufsetzbar ist. Gemäß einer weiteren Ausführungsart ist die horizontale Achse in Längsrichtung der Aufnahmeplatte und der Kippplatte ausgerichtet, d.h. parallel zur medianen Ebene eines Trägers der Einbeinschiene, dessen mit der zweiten Halteeinrichtung verbundener Fuß parallel zur Medianebene ausgerichtet ist. Die Kippplatte kippt den Rückfuß in seiner Außenrotationsbewegung in den Valgus. Bei der Bewegung des Fußes von der Innenrotation in die Außenrotation stellt sich die Ferse aufgrund ihrer Bewegungsanatomie von einer invertierten Position in eine Eversion ein. Um diese natürliche Bewegung nicht zu blockieren und eine anatomische Bewegung zuzulassen, wird die Einbeinschiene mit der Kippplatte versehen, welche passiv die Bewegung zulässt.

Gemäß einer weiteren Ausführungsart umfasst die Aufnahmeplatte ein rückwärtiges Teil und ein vorderes Teil, wobei das rückwärtige Teil und das vordere Teil über mindestens eine lösbare Verbindung miteinander verbindbar sind und das rückwärtige Teil und das vordere Teil jeweils ein Lager umfasst, in dem die horizontale Achse der Kippplatte gelagert ist.

Gemäß einer weiteren Ausführungsart sind dritte Drehwinkelbegrenzungen vorhanden, die ein Drehen der Kippplatte zwischen einer Neutralstellung eines auf die Kippplatte aufgesetzten und mit der zweiten Halteeinrichtung verbundenen Fußes an einem mit der ersten Halteeinrichtung verbundenen Bein und einer um maximal 10° bis 20°, vorzugsweise maximal 15° geschwenkten Stellung (Pronation) begrenzen. Durch die dritten Drehwinkelbegrenzungen wird die Bewegung der Kippplatte in pathologischer Richtung (Inversion) blockiert und die Pronation begrenzt.

Gemäß einer weiteren Ausführungsart ist die Kippplatte ausgewählt aus einer Range (Sortiment) von Kippplatten unterschiedlicher Größe für unterschiedlich große Füße. Hierdurch wird eine einfache Anpassung der Einbeinschiene an unterschiedliche Körpergrößen ermöglicht.

Gemäß einer weiteren Ausführungsart umfasst die zweite Halteeinrichtung mindestens einen zweiten Riemen mit einem zweiten Riemenverschluss und/oder mindestens eine Nut und/oder mindestens ein Profil und/oder mindestens ein Schnappelement zum lösbaren Halten eines Fußes oder eines gegebenenfalls mit einem zur Nut und/oder zum Profil und/oder Schnappelement komplementären Strukturelement versehenen Schuhes an der zweiten Halteeinrichtung.

Gemäß einer weiteren Ausführungsart umfasst das erste Drehgelenk ein Gabelteil und ein Gelenkunterteil. Das Gabelteil ist auf einfache Weise mit der ersten Halteeinrichtung verbindbar.

Gemäß einer weiteren Ausführungsart umfasst das Gelenkunterteil einen Gelenkkopf und einen damit verbundenen stangenförmigen Abschnitt mit einem Langloch und ist das Gelenkunterteil mit dem stangenförmigen Abschnitt in eine Führung mit einer Bohrung durch zwei einander gegenüberliegende Wände eingesetzt, in der eine das Langloch durchgreifende Schraube gehalten ist, die auf der einen Seite der Führung einen Schraubenkopf hat und auf der anderen Seite der Führung in ein Innengewinde eingreift, wodurch das Gelenkunterteil durch Festziehen der Schraube in verschiedenen Positionen in der Führung festklemmbar ist. Gemäß einer weiteren Ausführungsart ist die Führung ein Hohlprofil mit einem rechteckigen Querschnitt, in das der stangenförmige Abschnitt einschiebbar ist. Gemäß einer weiteren Ausführungsart ist die Führung zwischen den beiden einander gegenüberliegenden Wänden, die mit der Bohrung versehen sind, mit einem in Längsrichtung verlaufenden Schlitz versehen, wodurch das Verspannen der beiden Wände mit dem stangenförmigen Abschnitt durch Festziehen der Schraube erleichtert wird. Hierdurch ist eine Anpassung an verschiedene Körpergrößen und eine Fixierung der Einbeinschiene in der angepassten Stellung möglich.

Gemäß einer weiteren Ausführungsart weist das zweite Drehgelenk eine abgewinkelte Lagerplatte an der Führung auf, die durch eine seitliche Öffnung in eine Kammer der Aufnahmeplatte eingesetzt und darin um eine vertikale zweite Drehachse drehbar gelagert ist. Hierdurch wird eine platzsparende und konstruktiv einfache Ausbildung des zweiten Drehgelenkes ermöglicht.

Die Erfindung wird nachfolgend anhand der anliegenden Zeichnungen des Ausführungsbeispiels näher erläutert. In den Zeichnungen zeigen:
- Fig. 1: eine Einbeinschiene in einer Vorderansicht;
- Fig. 2: die Einbeinschiene in einer vergrößerten Detailansicht II. von Fig. 1;
- Fig. 3: die Einbeinschiene in einer Seitenansicht von der rechten Seite;
- Fig.4: die Einbeinschiene in einer vergrößerten Detailansicht IV von Fig. 3;
- Fig. 5: die Einbeinschiene in der Draufsicht;
- Fig. 6: die Einbeinschiene in einer vergrößerten Detailansicht VI von Fig. 5;
- Fig. 7: zweite Halteeinrichtung derselben Einbeinschiene in einem Sprengbild;
- Fig. 8: zweite Halteeinrichtung der Einbeinschiene in einer Unteransicht;
- Fig. 9: erstes Drehgelenk der Einbeinschiene in einem vergrößerten Sprengbild;
- Fig. 10: erstes Drehgelenk der Einbeinschiene in einem vergrößerten Teilschnitt,
- Fig. 11: zweites Drehgelenk der Einbeinschiene in einer vergrößerten Detailansicht von oben;
- Fig. 12: zweites Drehgelenk der Einbeinschiene in einer Perspektivansicht.

In der vorliegenden Anmeldung beziehen sich die Angaben "oben" und "unten" sowie "horizontal" und "vertikal" auf die Einbeinschiene, die mit dem ersten Drehgelenk oberhalb des zweiten Drehgelenkes angeordnet ist, wobei die erste Drehachse horizontal und die zweite Drehachse vertikal ausgerichtet ist.

Ferner gelten in dieser Anmeldung die anatomischen Bezeichnungen der Knochen und die standardisierten anatomischen Lage- und Richtungsbezeichnungen (z.B. wie in Abschnitt 3 der EP 2 637 612 B1 angegeben).

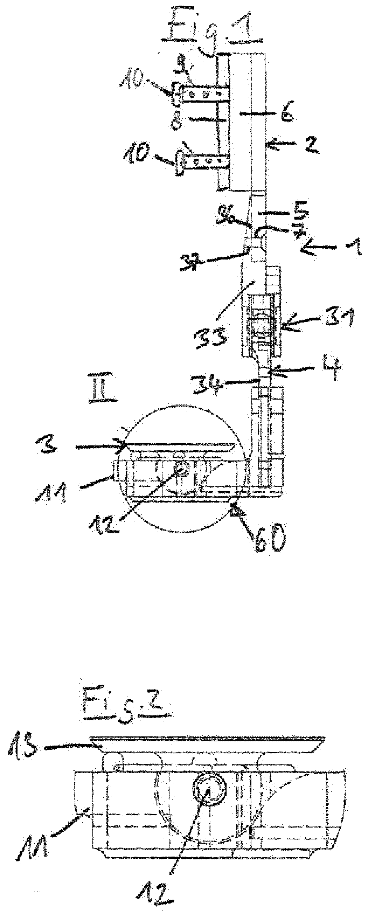

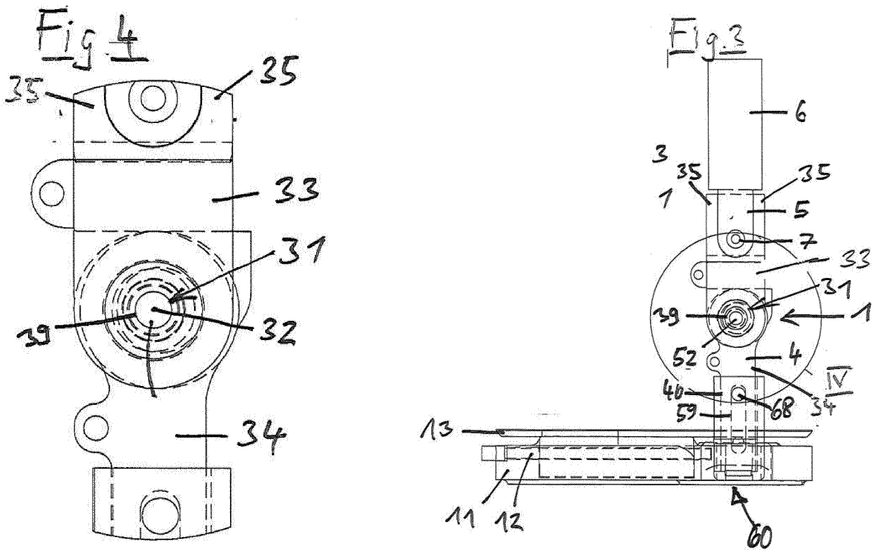

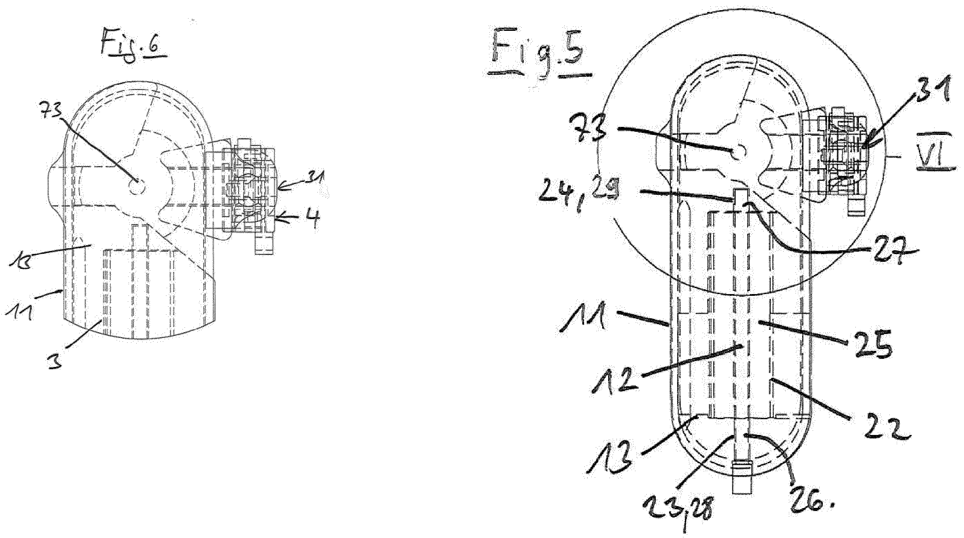

Die Einbeinschiene 1 umfasst gemäß Fig. 1 bis 6 eine erste Halteeinrichtung 2 zum lösbaren Verbinden mit einem Bein eines Trägers, eine zweite Halteeinrichtung 3 zum lösbaren Verbinden mit einem Fuß an demselben Bein des Trägers und eine Verbindungseinrichtung 4 zwischen der ersten Halteeinrichtung 2 und der zweiten Halteeinrichtung 3.

Die erste Halteeinrichtung 2 umfasst einen Haltezapfen 5 mit Rechteckquerschnitt und eine daran fixierte Schale 6, die ausgebildet ist, das Schienbein und zumindest einen Teil der Waden teilweise zu umschließen. Der Haltezapfen 5 hat in der Nähe seines unteren Endes ein erstes Befestigungsloch 7, das zum Befestigen der ersten Halteeinrichtung 2 an der Verbindungseinrichtung 4 dient. An der Innenseite der Schale 6 ist beispielsweise durch Ankleben eine Polsterung 8 fixiert. Die Polsterung 8 hat von den vertikalen Rändern der Schale 6 vorstehende Abschnitte, an denen außen Riemen 9 befestigt sind, die Schnallen, Löcher, Klettverschlüsse oder andere Elemente eines Riemenverschlusses 10 aufweisen. Wenn die Schale 6 an das Schienbein angesetzt ist, können die Riemen 9 um die Wade herumgelegt werden, um die erste Halteeinrichtung 2 am Bein des Trägers zu fixieren.

Die zweite Halteeinrichtung 3 umfasst eine Aufnahmeplatte 11 und eine darauf um eine horizontale Achse 12 kippbar gelagerte Kippplatte 13. Die Aufnahmeplatte 11 und die Kippplatte 13 haben in der Draufsicht etwa die Form einer Sohle eines Kinderschuhs.

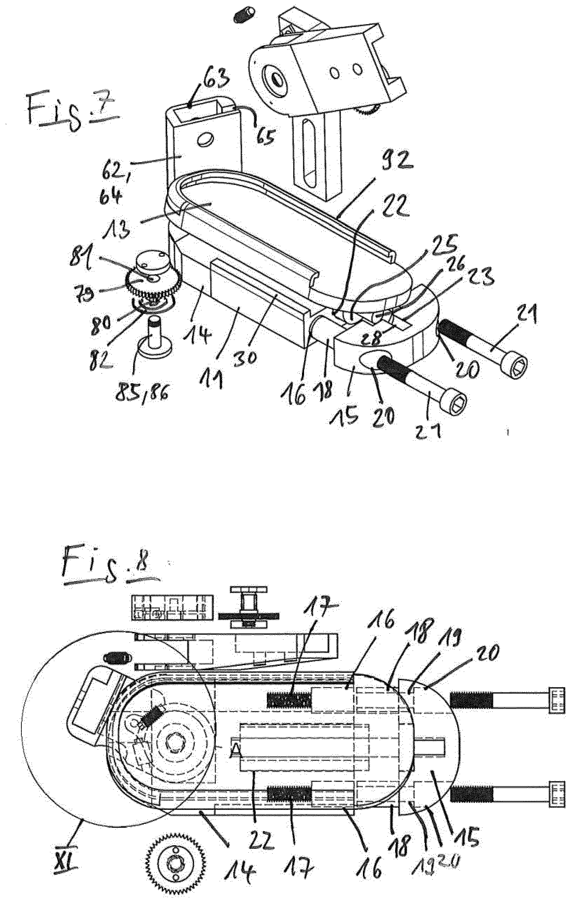

Die Aufnahmeplatte 11 umfasst gemäß Fig. 7 und 8 ein rückwärtiges Teil 14 und ein vorderes Teil 15, wobei das vordere Teil 15 in der Draufsicht die Form eines Kreissegments (z.B. eines Halbkreises) aufweist. Das rückwärtige Teil 14 weist an der vorderen Stirnfläche zwei parallele Sackbohrungen 16 auf, von deren Enden parallele erste Gewindebohrungen 17 ausgehen. Von der hinteren Stirnfläche des vorderen Teils 14 stehen zwei parallele Hülsen 18 vor, die in die Sackbohrungen 16 einsetzbar sind, wobei sie in den Sackbohrungen 16 geführt werden. Ferner weist das vordere Teil 15 zwei parallele Haltebohrungen 19 auf, die ausgehend von der kreisbogenförmig gekrümmten Vorderseite des vorderen Teils 15 sich bis zu den hinteren Enden der beiden Hülsen 18 erstrecken. Die beiden parallelen Haltebohrungen 19 haben vorn zwei parallele Erweiterungen 20. Das vordere Teil 15 ist mit den Hülsen 18 in die Sackbohrungen 16 eingesetzt und über zwei Befestigungsschrauben 21, die in die beiden Haltebohrungen 19 des vorderen Teils 15 eingesetzt sind und in die ersten Gewindebohrungen 17 des rückwärtigen Teiles 14 eingreifen, mit dem rückwärtigen Teil 14 verbunden. Die Köpfe der Befestigungsschrauben 19 sind in den Erweiterungen 20 der Haltebohrungen 19 aufgenommen. Hierdurch werden vorderes Teil 15 und rückwärtiges Teil 14 in einem bestimmten Abstand voneinander gehalten.

Die Aufnahmeplatte 11 umfasst eine erste Vertiefung 22 in Form eines Kreiszylindersegments. Diese erstreckt sich zwischen den parallelen Bohrungen 16, 19 über das vordere Teil 15 und das rückwärtige Teil 14 hinweg. Von den beiden Enden der ersten Vertiefung 22 gehen zweite Vertiefungen 23, 24 in Form von Kreiszylindersegmenten geringeren Durchmessers aus, von denen sich die eine im vorderen Teil 15 und die andere im rückwärtigen Teil 14 erstreckt.

Die Kippplatte 13 hat an der Unterseite ein vorstehendes Kreiszylindersegment 25. Das Kreiszylindersegment 25 nimmt mehr als die Hälfte eines vollständigen Zylinders ein. Von den beiden Stirnseiten des Kreiszylindersegments 25 stehen Lagerzapfen 26, 27 nach vorn und nach hinten vor.

Die Kippplatte 13 ist mit dem Kreiszylindersegment 25 in die erste Vertiefung 22 und mit den beiden Zapfen 26, 27 in die zweiten Vertiefungen 23, 24 eingesetzt und darin schwenkbar gelagert.

Die Kippplatte 13 wird dadurch an der Halteplatte gehalten, dass die Lagerzapfen 26, 27 von die zweiten Vertiefungen teilweise überspannenden Lagerschalen 28, 29 umschlossen werden.

An dem lateralen Rand steht von der Oberseite der Aufnahmeplatte 11 eine Anschlagleiste 30 nach oben vor.

Das Schwenken der Kippplatte 13 zur lateralen Seite hin (Eversion) wird durch die Anschlagleiste 30 begrenzt. Das Schwenken der Kippplatte 13 zur medianen Seite hin (Inversion) wird durch Anlage ihrer Unterseite an der Oberseite der Aufnahmeplatte 11 begrenzt.

Das Kreiszylindersegment 25 ist an seinem Außenumfang in der ersten Vertiefung 22 gelagert. Zwischen den Enden des Kreiszylindersegmentes 25 und der ersten Vertiefung 22 sind Spalte ausgebildet. Die Lagerzapfen 26, 27 sind zwischen den zweiten Vertiefungen 23, 24 und den Lagerschalen 28, 29 gelagert. Zwischen den Enden der Lagerzapfen 26, 27 und der zweiten Vertiefungen 23, 24 sind Spalte ausgebildet. Die Lagerung der Kippplatte 13 kann somit große Kräfte übertragen. Die Verlagerung der Kippplatte 13 in Längsrichtung wird durch die Spalte begrenzt.

Die Verbindungseinrichtung 4 umfasst gemäß Fig. 1 bis 6 ein erstes Drehgelenk 31 mit einer ersten Drehachse 32, die der Drehachse des oberen Sprunggelenkes eines Trägers der Einbeinschiene 1 entspricht. Das erste Drehgelenk 31 weist oben ein Gabelteil 33 und unten ein Gelenkunterteil 34 auf. Das Gabelteil 33 hat oben zwei Gabelzinken 35, die ausgebildet sind, den Haltezapfen 5 der ersten Halteeinrichtung 2 aufzunehmen. Die Gabelzinken 35 sind auf einer Seite durch eine Halteplatte 36 miteinander verbunden, die ein zweites Befestigungsloch 37 aufweist, das mit dem ersten Befestigungsloch 7 in dem Haltezapfen 5 korrespondiert, sodass die ersten und zweiten Befestigungslöcher 7, 37 zur Deckung kommen, wenn der Haltezapfen 5 zwischen die Gabelzinken 35 eingesetzt ist. Dann ist der Haltezapfen 5 mittels einer durch die ersten und zweiten Befestigungslöcher 7, 37 hindurchgeführten Schraube und einer auf diese aufgeschraubte Mutter an dem Gabelteil 33 fixierbar.

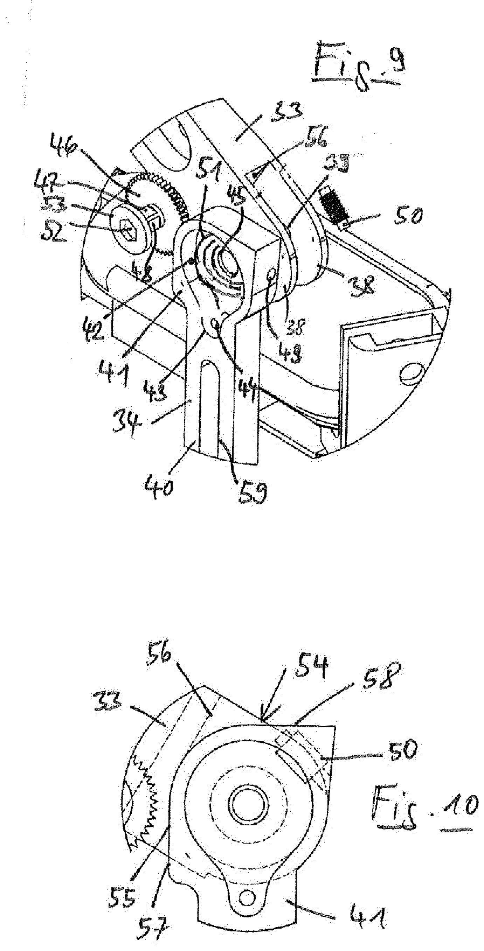

Das Gabelteil 33 weist gemäß Fig. 9 am unteren Ende zwei parallele Lagerplatten 38 auf, durch die horizontal erste Lagerbohrungen 39 hindurchverlaufen.

Das Gelenkunterteil 34 weist unten einen leistenförmigen Abschnitt 40 und am oberen Ende des leistenförmigen Abschnittes 40 einen Gelenkkopf 41 auf, der auf einer vertikalen Seite eine kreisscheibenförmige erste Vertiefung 42 aufweist, die unten eine glockenförmige erste Ausbuchtung 43 hat. In der ersten Ausbuchtung 43 steht ein horizontaler erster Zapfen 44 von der Basis der ersten Vertiefung 42 nach außen vor. Im Zentrum der ersten Vertiefung 42 hat der Gelenkkopf 41 eine erste Lagerbohrung 45. Der Gelenkkopf 41 ist zwischen die beiden Lagerplatten 38 des Gabelteiles 33 einschiebbar, sodass seine erste Lagerbohrung 45 mit den weiteren ersten Lagerbohrungen 39 des Gabelteiles 33 zur Deckung kommt.

In die erste Vertiefung 42 ist ein erstes Zahnrad 46 eingesetzt, das auf der von der Basis der ersten Vertiefung 42 abgewandten Seite einen von seinem Zentrum vorstehenden ersten Außenmehrkant 47 aufweist. Das erste Zahnrad 46 weist eine zentrale erste Lagerbohrung 48 auf, die sich durch den Außenmehrkant 47 hindurch erstreckt und mit der ersten Lagerbohrung 39 des Gelenkkopfes 41 fluchtet.

Der Gelenkkopf 41 weist eine erste Gewindebohrung 49 auf, die tangential zu der ersten Vertiefung 42 verläuft. In die erste Gewindebohrung 49 ist eine erste Madenschraube 50 eingesetzt, die an einem der Öffnung der ersten Gewindebohrung 49 nach außen zugewandten Ende einen Werkzeugangriff (z.B. Inbus) aufweist. Das Gewinde der Madenschraube 50 greift tangential in die erste Vertiefung 42 ein und steht mit der Zahnung am Außenumfang des ersten Zahnrades 46 in Eingriff.

In der ersten Vertiefung 42 ist eine erste Spiralfeder 51 angeordnet, die ein auf dem ersten Außenmehrkant 47 formschlüssig gehaltenes erstes Federende und ein auf dem ersten Zapfen 44 formschlüssig gehaltenes zweites Federende aufweist. Das erste Federende ist hierfür entsprechend der Form des Außenmehrkants 47 gebogen und das zweite Federende ist hierfür entsprechend der Form des Zapfens 44 ösenförmig gebogen.

Die erste Spiralfeder 51 ist so ausgebildet und angeordnet, dass sie bestrebt ist, das Gabelteil 33 nach vorn zu kippen.

Durch die ersten Lagerbohrungen 39, 45 des Gabelteiles 33 und des Gelenkunterteiles 34 ist eine erste Lagerschraube 52 hindurchgeführt, die auf der Außenseite der einen Lagerplatte 38 mit einem ersten Schraubenkopf 53 anliegt und in eine Mutter eingeschraubt ist, die an der Außenseite der anderen Lagerplatte 38 des Gabelteiles 33 anliegt. Die Lagerschraube 52 bildet die erste Drehachse 32.

Das erste Drehgelenk 31 umfasst erste Drehwinkelbegrenzungen 54, die eine erste und zweite Begrenzungsfläche 55, 56 zwischen den beiden Lagerplatten 38 des Gabelteiles 33 umfasst. Ferner umfassen die ersten Drehwinkelbegrenzungen 54 eine vertikale dritte Begrenzungsfläche 57 und eine horizontale vierte Begrenzungsfläche 58 am Gelenkkopf 41 des Gelenkunterteiles 34. Im Beispiel sind die Begrenzungsflächen 55, 56, 57, 58 so gewählt, dass bei Anlage der ersten Begrenzungsfläche 55 an der dritten Begrenzungsfläche 57 die Mittelachse des Gelenkteils 33 einen Winkel von 59,85° mit der Mittelachse des Gelenkunterteiles 45 einschließt. Beim Schwenken des Gabelteils 33, sodass die zweite Begrenzungsfläche 56 an der vierten Begrenzungsfläche 58 anliegt, sind beide Mittelachsen vertikal ausgerichtet und fallen zusammen.

Der leistenförmige Abschnitt 40 des Gelenkunterteiles 34 weist ein Langloch 59 auf, dessen Längsrichtung vertikal ausgerichtet ist.

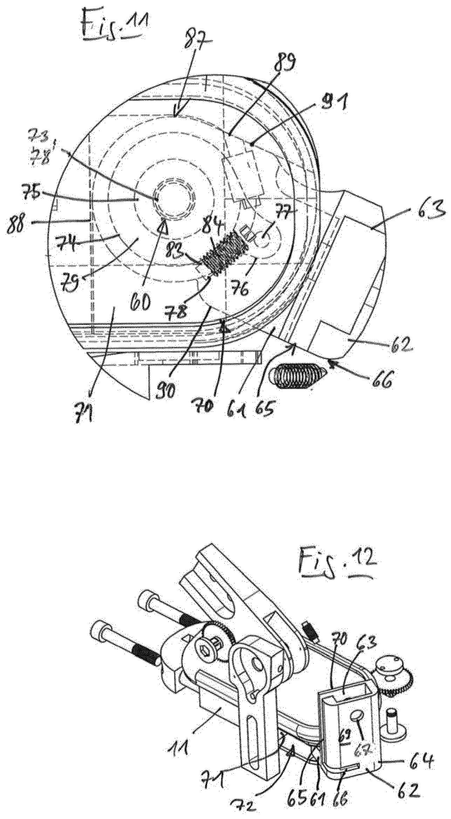

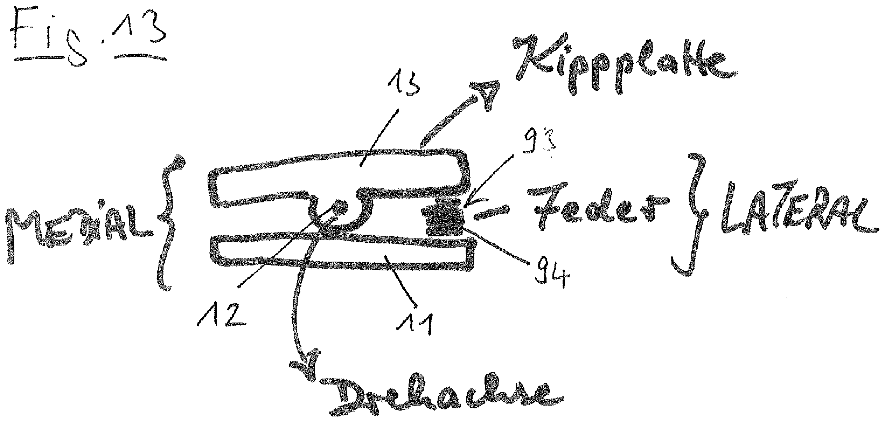

Ferner umfasst die Verbindungseinrichtung 4 ein zweites Drehgelenk 60, das gemäß Fig. 11 und 12 eine horizontale Lagerplatte 61 an einer Führung 62 mit einem vertikal ausgerichteten Führungskanal 63 in einem rechteckigen Hohlprofil 64 aufweist. Das Hohlprofil 64 weist an einem Rand einen vertikalen Schlitz 65 auf, der bis in den Führungskanal 63 reicht. Oberhalb der Lagerplatte 61 weist es auf einer Seite des vertikalen Schlitzes 65 einen horizontalen Schlitz 66 auf, der bis zur Mitte des Führungskanals 63 reicht. Ferner hat die Führung 62 auf beiden Seiten des Führungskanals 63 eine horizontale Bohrung 67.

Das Gelenkunterteil 34 ist mit dem leistenförmigen Abschnitt 40 in den Führungskanal 63 eingesetzt. Eine Schraube 68 ist durch die Bohrung 67 der Führung 62 und das Langloch 59 hindurchgeführt. Sie stützt sich mit einem Schraubenkopf auf einer Seite der Führung ab und ist am anderen Ende in ein Innengewinde einer Mutter eingeschraubt, die sie auf der gegenüberliegenden Außenseite der Führung 67 abstützt. Durch Festschrauben der Mutter sind zwei Seitenwände 69, 70 der Führung 62 geringfügig zusammenspannbar, wodurch das Gelenkunterteil 34 in der Führung 62 eingeklemmt wird. Das Zusammenspannen der Seitenwände 69, 70 wird durch die Schlitze 65, 66 erleichtert.

Die Aufnahmeplatte 11 weist eine Kammer 71 auf, die durch eine seitliche Öffnung 72 am medianen und hinteren Rand der Aufnahmeplatte 11 hindurch von außen zugänglich ist. Durch die seitliche Öffnung 72 hindurch greift die Lagerplatte 61 in die Kammer 71 ein. Die Lagerplatte 61 weist eine vertikale zweite Lagerbohrung 73 auf, welche senkrecht durch die Kammer 71 hindurch verläuft.

Die Lagerplatte 61 hat auf der Unterseite eine kreisscheibenförmige zweite Vertiefung 74. Zentral durch die Basis 75 der zweiten Vertiefung 74 hindurch verläuft die zweite Lagerbohrung 73. Die zweite Vertiefung 74 weist auf einer Seite eine glockenförmige zweite Ausbuchtung 76 auf, in der vertikal ein zweiter Zapfen 77 von der Basis 75 der zweiten Vertiefung 74 vorsteht.

Die Aufnahmeplatte 11 hat eine vertikale zweite Lagerbohrung 78, die sich unterhalb und oberhalb der Kammer 71 durch die diese begrenzenden Wände der Aufnahmeplatte 11 hindurch erstreckt.

In der zweiten Vertiefung 74 ist ein zweites Zahnrad 79 angeordnet, das an der Unterseite einen zentral vorstehenden zweiten Außenmehrkant 80 aufweist. Zentral durch das zweite Zahnrad 79 und den zweiten Außenmehrkant 80 hindurch erstreckt sich eine vertikale zweite Lagerbohrung 81.

Unterhalb des zweiten Zahnrades 79 ist um den zweiten Außenmehrkant 80 herum eine zweite Spiralfeder 82 angeordnet. Diese weist ein auf dem zweiten Außenmehrkant 80 formschlüssig gehaltenes drittes Federende und ein auf dem zweiten Zapfen 77 formschlüssig gehaltenes viertes Federende auf. Das dritte Federende ist entsprechend der Form des zweiten Außenmehrkantes 80 gebogen und das vierte Federende ist rund gebogen.

In der Lagerplatte 61 erstreckt sich tangential zur zweiten Vertiefung 74 eine zweite Gewindebohrung 83, in die eine zweite Madenschraube 84 eingesetzt ist. Die zweite Madenschraube 84 greift mit ihrem Schraubgewinde tangential in die zweite Vertiefung 74 ein und kämmt mit dem zweiten Zahnrad 79. Die zweite Madenschraube 84 hat an einem der Öffnung der zweiten Gewindebohrung 83 nach außen zugewandten Ende einen Werkzeugangriff (z.B. Inbus).

In die zweiten Lagerbohrungen 73, 78, 81 ist von unten eine zweite Lagerschraube 85 eingesetzt, welche die zweiten Lagerbohrungen 75, 78, 81 durchgreift und in eine an der Oberseite der Aufnahmeplatte 11 anliegende Mutter eingeschraubt ist. Der Kopf der Schraube und die Mutter sind in Vertiefungen der Aufnahmeplatte 11 angeordnet, sodass sie nicht nach außen vorstehen. Die zweite Lagerschraube 85 bildet eine zweite Drehachse 86.

Das zweite Drehgelenk 60 umfasst gemäß Fig. 11 zweite Drehwinkelbegrenzungen 87. Diese werden gebildet von fünften und sechsten Begrenzungsflächen 88, 89 der Kammer 71, wobei die fünfte Begrenzungsfläche 88 einen Winkel von 90° mit der Längsachse der Aufnahmeplatte 11 (und damit mit der Medianebene) und die sechste Begrenzungsfläche 89 einen Winkel von 35° zur Längsachse der Aufnahmeplatte 11 (und damit zu Medianebene) einschließt. Die Lagerplatte 61 weist am äußeren Rand eine siebte Begrenzungsfläche 90 und eine achte Begrenzungsfläche 91 auf. Diese sind so orientiert, dass bei Anlage der fünften Begrenzungsfläche 88 an der siebten Begrenzungsfläche 90 die Aufnahmeplatte 11 mit einem darauf angeordneten Fuß parallel zur Medianebene ausgerichtet ist. Bei Anlage der sechsten Begrenzungsfläche 89 an der achten Begrenzungsfläche 91 ist der Fuß um 35° nach außen rotiert, wie in Fig. 11 gezeigt. Hierdurch wird das Schwenken der Lagerplatte 61 in beiden Richtungen begrenzt. Die zweite Spiralfeder 82 ist so ausgebildet und angeordnet, dass sie die Aufnahmeplatte 11 in die nach außen rotierte Stellung belastet. Mittels der zweiten Madenschraube 84 ist die Vorspannung der zweiten Spiralfeder 82 einstellbar.

Die Kippplatte 13 weist gemäß Fig. 7 am lateralen, medialen und hinteren Rand L-förmige Profilschienen 92 auf, welche die Kippplatte 13 mit ihren abgewickelten Enden teilweise übergreifen. Von der Vorderseite der Kippplatte 13 aus ist ein Schuh mit einem entsprechend ausgebildeten Rand der Sohle auf die Kippplatte 13 aufschiebbar. In der aufgeschobenen Stellung wird der vorspringende Rand von den Profilschienen 92 übergriffen. In dieser Stellung kann der Schuh durch eine zusätzlichen Schnappverbindung oder durch einen Klickverschluss gesichert werden.

Die Einbeinschiene 1 kann angelegt werden, indem ein Schuh mit der Kippplatte 13 verbunden und die Schale 6 mit den Riemen 9 an der Wade des Kindes befestigt wird. Durch die Spiralfedern 51, 82 wird der Fuß des Trägers in Richtung einer Dorsalreflexion und in Richtung einer Außenrotation belastet. Die Drehwinkelbegrenzungen 54, 87 verhindern, dass der Fuß in den pathologischen Bereich bewegt wird und in die Klumpfußstellung gelangt. Hierdurch wird eine Dorsalextension und eine Außenrotation im therapeutischen Bereich erreicht.

Eine Anpassung der Einbeinschiene 1 an verschiedene Körpergrößen ist durch Austausch der Kippplatte 13 und/oder durch Austausch der ersten Halteeinrichtung 2 möglich. Hierfür kann ein Sortiment aus Kippplatten 13 unterschiedlicher Größe und/oder Form und ein Sortiment aus ersten Halteeinrichtungen 2 mit Schalen 6 und Riemen 9 unterschiedlicher Größe und/oder Form vorgehalten werden.

### Bezugszeichenliste

- 1: Einbeinschiene
- 2: erste Halteeinrichtung
- 3: zweite Halteeinrichtung
- 4: Verbindungseinrichtung
- 5: Haltezapfen
- 6: Schale
- 7: erstes Befestigungsloch
- 8: Polsterung
- 9: Riemen
- 10: Riemenverschluss
- 11: Aufnahmeplatte
- 12: Achse
- 13: Kippplatte
- 14: rückwärtiges Teil
- 15: vorderes Teil
- 16: Sackbohrung
- 17: erste Gewindebohrung
- 18: Hülsen
- 19: Haltebohrung
- 20: Erweiterung
- 21: Befestigungsschraube
- 22: erste Vertiefung
- 23, 24: zweite Vertiefung
- 25: Kreiszylindersegment
- 26, 27: Lagerzapfen
- 28, 29: Lagerschale
- 30: Anschlagleiste
- 31: erstes Drehgelenk
- 32: erste Drehachse
- 33: Gabelteil
- 34: Gelenkunterteil
- 35: Gabelzinken
- 36: Halteplatte
- 37: zweite Befestigungslöcher
- 38: Lagerplatte
- 39: erste Lagerbohrung
- 40: leistenförmiger Abschnitt
- 41: Gelenkkopf
- 42: erste Vertiefung
- 43: erste Ausbuchtung
- 44: erster Zapfen
- 45: erste Lagerbohrung
- 46: erstes Zahnrad
- 47: erster Außenmehrkant
- 48: erste Lagerbohrung
- 49: erste Gewindebohrung
- 50: erste Madenschraube
- 51: erste Spiralfeder
- 52: erste Lagerschraube
- 53: erster Schraubenkopf
- 54: erste Drehwinkelbegrenzungen
- 55: erste Begrenzungsfläche
- 56: zweite Begrenzungsfläche
- 57: dritte Begrenzungsfläche
- 58: vierte Begrenzungsfläche
- 59: Langloch
- 60: zweites Drehgelenk
- 61: Lagerplatte
- 62: Führung
- 63: Führungskanal
- 64: Hohlprofil
- 65: vertikaler Schlitz
- 66: horizontaler Schlitz
- 67: horizontale Bohrung
- 68: Schraube
- 69, 70: Seitenwände
- 71: Kammer
- 72: Öffnung
- 73: zweite Lagerbohrung
- 74: zweite Vertiefung
- 75: Basis
- 76: zweite Ausbuchtung
- 77: zweiter Zapfen
- 78: zweite Lagerbohrung
- 79: zweites Zahnrad
- 80: zweiter Außenmehrkant
- 81: zweite Lagerbohrung
- 82: zweite Spiralfeder
- 83: zweite Gewindebohrung
- 84: zweite Madenschraube
- 85: zweite Lagerschraube
- 86: zweite Drehgelenkachse
- 87: zweite Drehwinkelbegrenzungen
- 88: fünfte Begrenzungsfläche
- 89: sechste Begrenzungsfläche
- 90: siebte Begrenzungsfläche
- 91: achte Begrenzungsfläche
- 92: Profilschiene

## Patentansprüche

1. Einbeinschiene (1) zur Behandlung von Klumpfüßen und anderen Fußdeformitäten umfassend eine erste Halteeinrichtung (2) zum lösbaren Verbinden mit einem Bein eines Trägers, eine zweite Halteeinrichtung (3) zum lösbaren Verbinden mit einem Fuß an demselben Bein des Trägers und eine Verbindungseinrichtung (4) zwischen der ersten Halteeinrichtung und der zweiten Halteeinrichtung, wobei die Verbindungseinrichtung ein erstes Drehgelenk (31) mit einer ersten Drehachse (32) umfasst und die Verbindungseinrichtung ein zweites Drehgelenk (60) mit einer vertikal ausgerichteten zweiten Drehachse (86) umfasst, wodurch das erste Drehgelenk den Fuß eines Trägers bei einer Dorsalextension und das zweite Drehgelenk den Fuß des Trägers bei einer Außenrotation führt, **dadurch gekennzeichnet, dass** die erste Drehachse der Drehachse des oberen Sprunggelenkes entspricht.

2. Einbeinschiene nach Anspruch 1, bei der das erste Drehgelenk eine vorgespannte erste Federeinrichtung (51.1) umfasst, die das erste Drehgelenk in Richtung der Ausführung einer Dorsalextension eines mit der zweiten Halteeinrichtung verbundenen Fußes belastet und/oder bei der das zweite Drehgelenk eine vorgespannte zweite Federeinrichtung (82.1) umfasst, die das zweite Drehgelenk in Richtung der Ausführung einer Außenrotation eines mit der zweiten Halteeinrichtung verbundenen Fußes belastet.

3. Einbeinschiene nach Anspruch 2, bei der die erste Federeinrichtung eine erste Federeinrichtung mit einstellbarer Vorspannung und/oder bei der die zweite Federeinrichtung eine zweite Federeinrichtung mit einstellbarer Vorspannung ist.

4. Einbeinschiene nach Anspruch 2 oder 3, bei der die erste Federeinrichtung eine erste Spiralfeder (51) und/oder bei der die zweite Federeinrichtung eine zweite Spiralfeder (82) ist.

5. Einbeinschiene nach Anspruch 3 und 4, bei der das erste Drehgelenk ein erstes Zahnrad : (46) mit einem damit verbunden zentralen ersten Außenmehrkant (47) und einer ersten Lagerbohrung (39) eine in die erste Lagerbohrung und in weitere erste Lagerbohrungen der beiden Gelenkteile des ersten Drehgelenks eingesetzte erste Drehachse (32) umfasst, die erste Spiralfeder ein auf dem ersten Außenmehrkant formschlüssig gehaltenes erstes Federende und ein an einem ersten Gelenkteil gehaltenes zweites Federende aufweist und in eine erste Gewindebohrung (17) in dem anderen ersten Gelenkteil eine mit dem ersten Zahnrad kämmende erste Madenschraube (50) mit einem ersten Werkzeugangriff eingeschraubt ist, wodurch mittels eines an den ersten Werkzeugangriff angesetzten Werkzeugs das erste Zahnrad drehbar und die erste Spiralfeder in unterschiedlichem Ausmaß vorspannbar ist und/oder bei der das zweite Drehgelenk ein zweites Zahnrad (79) mit einem damit verbundenen zentralen zweiten Außenmehrkant (80) und einer zweiten Lagerbohrung (73) eine in die zweite Lagerbohrung und in weitere zweite Lagerbohrungen der beiden zweiten Gelenkteile des zweiten Drehgelenkes eingesetzte zweite Drehachse umfasst, die zweite Spiralfeder ein auf dem zweiten Außenmehrkant formschlüssig gehaltenes erstes Federende und ein an einem zweiten Gelenkteil gehaltenes zweites Federende aufweist und in eine zweite Gewindebohrung (83) in dem anderen zweiten Gelenkteil in eine mit dem zweiten Zahnrad kämmende zweite Madenschraube (84) mit einem zweiten Werkzeugangriff eingeschraubt ist, wodurch mittels eines an den zweiten Werkzeugangriff angesetzten Werkzeugs das zweite Zahnrad drehbar und die zweite Spiralfeder in unterschiedlichem Ausmaß vorspannbar ist.

6. Einbeinschiene nach einem der Ansprüche 1 bis 5, bei der das erste Drehgelenk erste Drehwinkelbegrenzungen (54) umfasst, welche ein Drehen des ersten Drehgelenkes begrenzen, sodass ein mit der zweiten Halteeinrichtung verbundener Fuß an einem mit der ersten Halteeinrichtung verbundenen Bein zwischen einer Neutralstellung und einer Dorsalflektion mit einem Winkel von maximal 55 bis 30°, vorzugsweise von maximal 45° drehbar ist und/oder das zweite Drehgelenk zweite Drehwinkelbegrenzungen (87) umfasst, welche die Drehbewegung des zweiten Drehgelenkes begrenzen, sodass ein mit der zweiten Halteeinrichtung verbundener Fuß an einem mit der ersten Halteeinrichtung verbundenen Bein zwischen einer Stellung parallel zur Medianebene und einer Abduktion mit einem Winkel von maximal 75 bis 50°, vorzugsweise von maximal 65° zur Medianebene bewegbar ist.

7. Einbeinschiene nach einem der Ansprüche 2 bis 6, die eine erste Feststelleinrichtung und eine zweite Feststelleinrichtung aufweist, die ausgebildet sind, die erste Federeinrichtung und die zweite Federeinrichtung in Positionen festzustellen, in denen die erste Halteeinrichtung und die zweite Halteeinrichtung ein Bein und den zugehörigen Fuß in der Normalstellung halten.

8. Einbeinschiene nach einem der Ansprüche 1 bis 7, bei der die erste Halteeinrichtung eine das Schienbein und zumindest einen Teil der Waden einfassende Schale (6) aufweist.

9. Einbeinschiene nach einem der Ansprüche 1 bis 8, bei der die zweite Halteeinrichtung eine Aufnahmeplatte (11) zum Unterstützen des Fußes aufweist.

10. Einbeinschiene nach einem der Ansprüche 1 bis 9, bei der die zweite Halteeinrichtung oberhalb der Aufnahmeplatte eine an der Aufnahmeplatte um eine horizontale Achse kippbar gelagerte Kippplatte (13) umfasst, auf der der Fuß aufsetzbar ist.

11. Einbeinschiene nach Anspruch 10, bei der die Aufnahmeplatte ein rückwärtiges Teil (14) und ein vorderes Teil (15) umfasst, wobei das rückwärtige Teil und das vordere Teil über mindestens eine lösbare Verbindung miteinander verbindbar sind und das rückwärtige Teil und das vordere Teil jeweils ein Lager umfasst, in dem die horizontale Achse der Kippplatte gelagert ist.

12. Einbeinschiene nach einem der Ansprüche 8 bis 11, bei der die Schale ausgewählt ist aus einer Range (Sortiment) von Schalen unterschiedlicher Größe für unterschiedliche Körpergrößen und/oder die Kippplatte ausgewählt ist aus einer Range (Sortiment) von Kippplatten unterschiedlicher Größe für unterschiedlich große Füße.

13. Einbeinschiene nach einem der Ansprüche 10 bis 12, die eine dritte Drehwinkelbegrenzungen aufweist, die ein Drehen der Kippplatte zwischen einer Neutralstellung eines auf die Kippplatte aufgesetzten und mit der zweiten Halteeinrichtung verbundenen Fußes an einem mit der ersten Halteeinrichtung verbundenen Bein und einer um maximal 10° bis 20°, vorzugsweise maximal 15° geschwenkten Stellung begrenzen.

14. Einbeinschiene nach einem der Ansprüche 1 bis 13, bei der das erste Drehgelenk ein Gabelteil (33) und ein Gelenkunterteil (34) umfassen.

15. Einbeinschiene nach Anspruch 14, bei der das Gelenkunterteil einen Gelenkkopf (41) und einen damit verbundenen stangenförmigen Abschnitt (40) mit einem Langloch (59) umfasst und mit dem stangenförmigen Abschnitt in eine Führung (62) mit einer Bohrung durch zwei einander gegenüberliegende Wände eingesetzt ist, in der eine das Langloch durchgreifende Schraube (68) gehalten ist, die auf der einen Seite der Führung einen Schraubenkopf hat und auf der anderen Seite der Führung in ein Innengewinde eingreift, wodurch das Gelenkunterteil durch Festziehen der Schraube in verschiedenen Positionen in der Führung festklemmbar ist.

16. Einbeinschiene nach einem der Ansprüche 1 bis 15, bei der das zweite Drehgelenk eine abgewinkelte Lagerplatte (61) an der Führung aufweist, die durch eine seitliche Öffnung (72) in eine Kammer (71) der Aufnahmeplatte eingesetzt und darin um eine vertikale zweite Drehachse drehbar gelagert ist.

## Claims

1. A unilateral orthotic (1) for the treatment of club foot and other foot deformities, comprising a first holding apparatus (2) for detachable connection to a leg of a wearer, a second holding apparatus (3) for detachable connection to a foot on the same leg of the wearer, and a connecting apparatus (4) between the first holding apparatus and the second holding apparatus, wherein the connecting apparatus comprises a first rotational joint (31) having a first axis of rotation (32) and the connecting apparatus comprises a second rotational joint (60) having a vertically aligned second axis of rotation (86), as a result of which the first rotational joint guides the foot of a wearer during a dorsal extension and the second rotational joint guides the foot of the wearer during an external rotation, **characterized in that** the first axis of rotation (32) corresponds to the axis of rotation of the upper ankle joint.

2. The unilateral orthotic according to Claim 1, in which the first rotational joint comprises a biased first spring apparatus (51.1) which loads the first rotational joint in the direction of the execution of a dorsal extension of a foot connected to the second holding apparatus and/or in which the second rotational joint comprises a biased second spring apparatus (82.1) which loads the second rotational joint in the direction of the execution of an external rotation of a foot connected to the second holding apparatus.

3. The unilateral orthotic according to Claim 2, in which the first spring apparatus is a first spring apparatus having an adjustable bias and/or in which the second spring apparatus is a second spring apparatus having an adjustable bias.

4. The unilateral orthotic according to Claim 2 or 3, in which the first spring apparatus is a first spiral spring (51) and/or in which the second spring apparatus is a second spiral spring (82).

5. The unilateral orthotic according to Claim 3 and 4, in which the first rotational joint comprises a first gear wheel (46) having a central first external polygon (47) connected thereto and a first bearing bore (39), a first axis of rotation (32) inserted into the first bearing bore and into further first bearing bores of the two joint parts of the first rotational joint, the first spiral spring has a first spring end positively held on the first external polygon, and a second spring end held on a first joint part and a first locking screw (50), which meshes with the first gear wheel and which has a first tool engagement portion, is screwed into a first threaded bore (17) in the other first joint part, as a result of which, by means of a tool applied to the first tool engagement portion, the first gear wheel can be rotated and the first spiral spring can be biased to different extents and/or in which the second rotational joint comprises a second gear wheel (79) having a central second external polygon (80) connected thereto and a second bearing bore (73), a second axis of rotation inserted into the second bearing bore and into further second bearing bores of the two second joint parts of the second rotational joint, the second spiral spring has a first spring end positively held on the second external polygon and a second spring end held on a second joint part and a second locking screw (84), which meshes with the second gear wheel and which has a second tool engagement portion, is screwed into a second threaded bore (83) in the other second joint part, as a result of which, by means of a tool applied to the second tool engagement portion, the second gear wheel can be rotated and the second spiral spring can be biased to different extents.

6. The unilateral orthotic according to any one of Claims 1 to 5, in which the first rotational joint comprises first rotational angle delimitations (54) which delimit a rotation of the first rotational joint so that a foot connected to the second holding apparatus can be rotated at a leg connected to the first holding apparatus between a neutral position and a dorsal flection with an angle of a maximum of 55 to 30°, preferably a maximum of 45°, and/or the second rotational joint comprises second rotational angle delimitations (87) which delimit the rotational movement of the second rotational joint so that a foot connected to the second holding apparatus can be moved at a leg connected to the first holding apparatus between a position parallel to the median plane and an abduction having an angle of a maximum of 75 to 50°, preferably a maximum of 65° to the median plane.

7. The unilateral orthotic according to any one of Claims 2 to 6, which has a first locking apparatus and a second locking apparatus which are configured to lock the first spring apparatus and the second spring apparatus in positions in which the first holding apparatus and the second holding apparatus hold a leg and the associated foot in the normal position.

8. The unilateral orthotic according to any one of Claims 1 to 7, in which the first holding apparatus has a bowl (6) which encompasses the shin and at least a part of the calves.

9. The unilateral orthotic according to any one of Claims 1 to 8, in which the second holding apparatus has a receiving plate for supporting the foot.

10. The unilateral orthotic according to any one of Claims 1 to 9, in which the second holding apparatus above the receiving plate comprises a tilting plate (13) which is tiltably mounted on the receiving plate about a horizontal axis, on which tilting plate the foot can be placed.

11. The unilateral orthotic according to Claim 10, in which the receiving plate comprises a rear part (14) and a front part (15), wherein the rear part and the front part can be connected to one another via at least one detachable connection and the rear part and the front part respectively comprise a bearing, in which the horizontal axis of the tilting plate is mounted.

12. The unilateral orthotic according to any one of Claims 8 to 11, in which the bowl is selected from a range (assortment) of bowls of different sizes for different body sizes and/or the tilting plate is selected from a range (assortment) of tilting plates of different sizes for different sized feet.

13. The unilateral orthotic according to any one of Claims 10 to 12, which has third rotational angle delimitations which delimit a rotation of the tilting plate between a neutral position of a foot placed on the tilting plate and connected to the second holding apparatus at a leg connected to the first holding apparatus and a position swiveled by a maximum of 10° to 20°, preferably by a maximum of 15°.

14. The unilateral orthotic according to any one of Claims 1 to 13, in which the first rotational joint comprises a fork part (33) and a lower joint part (34).

15. The unilateral orthotic according to Claim 14, in which the lower joint part comprises a joint head (41) and a bar-shaped section (40) which is connected thereto and which has an elongated bore (59) and is inserted with the bar-shaped section into a guide (62) with a bore through two walls located opposite one another, in which a screw (68) which engages through the elongated bore is held, which screw has a screw head on the one side of the guide and engages in an inner thread on the other side of the guide, as a result of which the joint lower part can be firmly clamped in various positions in the guide by tightening the screw.

16. The unilateral orthotic according to any one of Claims 1 to 15, in which the second rotational joint has an angled bearing plate (61) on the guide, which is inserted through a lateral opening (72) into a chamber (71) of the receiving plate and is rotatably mounted therein about a vertical second axis of rotation.

## Revendications

1. Gouttière de jambe destinée au traitement des pieds-bots et d'autres difformités du pied, comportant un premier appareil de maintien (2) destiné à être relié de façon détachable à une jambe d'un porteur, un deuxième appareil de maintien (3) destiné à être relié de façon détachable à un pied sur la même jambe du porteur et un appareil de liaison (4) entre le premier appareil de maintien et le deuxième appareil de maintien, dans laquelle l'appareil de liaison comporte un premier joint rotatif (31) avec un premier axe de rotation (32) et l'appareil de liaison comporte un deuxième joint rotatif (60) avec un deuxième axe de rotation (86) orienté verticalement,
moyennant quoi le premier joint rotatif guide le pied d'un porteur lors d'une extension dorsale et le deuxième joint rotatif guide le pied du porteur lors d'une rotation externe,
**caractérisée en ce que** le premier axe de rotation correspond à l'axe de rotation de la partie supérieure de la cheville.

2. Gouttière de jambe selon la revendication 1, dans laquelle le premier joint rotatif comporte un premier appareil à ressort (51.1) précontraint, lequel sollicite le premier joint rotatif dans une direction d'exécution d'extension dorsale d'un pied relié au deuxième appareil de maintien et/ou dans laquelle le deuxième joint rotatif comporte un deuxième appareil à ressort (82.1) précontraint, lequel sollicite le deuxième joint rotatif dans une direction d'exécution de rotation externe d'un pied relié au deuxième appareil de maintien.

3. Gouttière de jambe selon la revendication 2, dans laquelle le premier appareil à ressort est un premier appareil à ressort avec une précontrainte réglable et/ou dans laquelle le deuxième appareil à ressort est un deuxième appareil à ressort avec une précontrainte réglable.

4. Gouttière de jambe selon la revendication 2 ou 3, dans laquelle le premier appareil à ressort est un premier ressort hélicoïdal (51) et/ou dans laquelle le deuxième appareil à ressort est un deuxième ressort hélicoïdal (82).

5. Gouttière de jambe selon la revendication 3 ou 4, dans laquelle le premier joint rotatif comporte une première roue dentée (46) dotée d'un premier polygone externe central (47) relié à celle-ci et d'un premier alésage de palier (39), un premier axe de rotation (32) inséré dans le premier alésage de palier et dans d'autres premiers alésages de palier des deux parties de joint du premier joint rotatif, le premier ressort hélicoïdal présente une première extrémité de ressort maintenue par complémentarité de forme sur le premier polygone externe et une deuxième extrémité de ressort maintenue sur une première partie de joint, et une première vis sans tête (50) dotée d'une première prise d'outil, engrenant avec la première roue dentée, est vissée dans un premier alésage fileté (17) dans l'autre première partie de joint, moyennant quoi la première roue dentée peut tourner et le premier ressort hélicoïdal peut être précontraint selon un degré variable, au moyen d'un outil placé sur la première prise d'outil, et/ou dans laquelle le deuxième joint rotatif comporte une deuxième roue dentée (79) dotée d'un deuxième polygone externe central (80) relié à celle-ci et d'un deuxième alésage de palier (73), un deuxième axe de rotation inséré dans le deuxième alésage de palier et dans d'autres deuxièmes alésages de palier des deux deuxièmes parties de joint du deuxième joint rotatif, le deuxième ressort hélicoïdal présente une première extrémité de ressort maintenue par complémentarité de forme sur le deuxième polygone externe et une deuxième extrémité de ressort maintenue sur une deuxième partie de joint, et une deuxième vis sans tête (84) dotée d'une deuxième prise d'outil, engrenant avec la deuxième roue dentée, est vissée dans un deuxième alésage fileté (83) dans l'autre deuxième partie de joint, moyennant quoi la deuxième roue dentée peut tourner et le deuxième ressort hélicoïdal peut être précontraint selon un degré variable, au moyen d'un outil placé sur la deuxième prise d'outil.

6. Gouttière de jambe selon l'une des revendications 1 à 5, dans laquelle le premier joint rotatif comporte des premières limitations d'angle de rotation (54), lesquelles limitent une rotation du premier joint rotatif, de sorte qu'un pied relié au deuxième appareil de maintien sur une jambe reliée au premier appareil de maintien peut tourner entre une position neutre et une flexion dorsale selon un angle maximal de 55 à 30°, de préférence un angle maximal de 45°, et/ou le deuxième joint rotatif comporte des deuxièmes limitations d'angle de rotation (87), lesquelles limitent une rotation du deuxième joint rotatif, de sorte qu'un pied relié au deuxième appareil de maintien sur une jambe reliée au premier appareil de maintien peut tourner entre une position parallèle au plan médian et une abduction selon un angle maximal de 75 à 50°, de préférence un angle maximal de 65° par rapport au plan médian.

7. Gouttière de jambe selon l'une des revendications 2 à 6, laquelle présente un premier appareil de blocage et un deuxième appareil de blocage, lesquels sont conçus pour bloquer le premier appareil à ressort et le deuxième appareil à ressort dans des positions dans lesquelles le premier appareil de maintien et le deuxième appareil de maintien maintiennent une jambe et le pied correspondant dans la position normale.

8. Gouttière de jambe selon l'une des revendications 1 à 7, dans laquelle le premier appareil de maintien présente une coquille (6) entourant le tibia et une partie au moins des mollets.

9. Gouttière de jambe selon l'une des revendications 1 à 8, dans laquelle le deuxième appareil de maintien présente une plaque de réception (11) destinée à soutenir le pied.

10. Gouttière de jambe selon l'une des revendications 1 à 9, dans laquelle, au-dessus de la plaque de réception, le deuxième appareil de maintien comporte une plaque pivotante (13) montée sur la plaque de réception de manière à pouvoir pivoter autour d'un axe horizontal, sur laquelle le pied peut être posé.

11. Gouttière de jambe selon la revendication 10, dans laquelle la plaque de réception comporte une partie arrière (14) et une partie avant (15), dans laquelle la partie arrière et la partie avant sont reliées entre elles par au moins une liaison détachable et la partie arrière et la partie avant comportent respectivement un palier, dans lequel est monté l'axe horizontal de la plaque pivotante.

12. Gouttière de jambe selon l'une des revendications 8 à 11, dans laquelle la coquille est sélectionnée parmi une gamme (assortiment) de coquilles de différentes tailles pour différentes corpulences, et/ou la plaque pivotante est sélectionnée parmi une gamme (assortiment) de plaques pivotantes de différentes tailles pour différentes tailles de pied.

13. Gouttière de jambe selon l'une des revendications 10 à 12, laquelle présente des troisièmes limitations d'angle de rotation, lesquelles limitent une rotation de la plaque pivotante entre une position neutre d'un pied posé sur la plaque pivotante et relié au deuxième appareil de maintien sur une jambe reliée au premier appareil de maintien et une position pivotée de 10° à 20° maximum, de préférence de 15° maximum.

14. Gouttière de jambe selon l'une des revendications 1 à 13, dans laquelle le premier joint rotatif comporte une partie fourchue (33) et une partie inférieure de joint (34).

15. Gouttière de jambe selon la revendication 14, dans laquelle la partie inférieure de joint comporte une tête de joint (41) et une section en forme de barre (40) reliée à celle-ci avec un trou oblong (59) et est insérée avec la section en forme de barre dans un guidage (62) doté d'un alésage à travers deux parois opposées l'une à l'autre, dans lequel est maintenue une vis (68) traversant le trou oblong, laquelle possède une tête de vis sur un côté du guidage et s'engage dans un filetage intérieur sur l'autre côté du guidage, moyennant quoi la partie inférieure de joint peut être serrée dans le guidage dans différentes positions par serrage de la vis.

16. Gouttière de jambe selon l'une des revendications 1 à 15, dans laquelle le deuxième joint rotatif présente une plaque d'appui coudée (61) sur le guidage, laquelle est introduite dans une chambre (71) de la plaque de réception par une ouverture latérale (72) et montée dans celle-ci de manière à pouvoir tourner autour d'un deuxième axe de rotation vertical.
